# EUROPEAN PATENT SPECIFICATION

(11) **EP 1 384 478 B1**
(45) Date of publication and mention of the grant of the patent: **16.03.2011**
(21) Application number: 03020587.6
(22) Date of filing: 29.09.1999
(51) Int. Cl.: A61K 45/06, A61K 31/57, A61K 31/4709, A61K 31/5383

(54) **ANTIBIOTIC COMPOSITIONS CONTAINING MOXIFLOXACIN FOR TREATMENT OF THE EYE**
ANTIBIOTISCHE ZUSAMMENSETZUNG, DIE MOXIFLOXACIN ENTHÄLT, ZUR BEHANDLUNG VON AUGEN
COMPOSITIONS ANTIBIOTIQUES CONTENANT MOXIFLOXACIN DESTINEES AU TRAITEMENT DES YEUX

(30) Priority: 30.09.1998 US 102504 P; 30.09.1998 US 102506 P
(43) Date of publication of application: 28.01.2004
(62) Divisional of application: 99956504.7
(73) Proprietor: Alcon, Inc., 6331 Hünenberg (CH)
(72) Inventor: Cagle, Gerald, Forth Worth, TX 76116 (US); Abshire, Robert L., Forth Worth, TX 76116 (US); Stromann, David W., Irving, TX 75063 (US); Yanni, John M., Burleson, TX 76028 (US)
(74) Representative: Best, Michael

(56) References cited:
- EP-A- 0 550 903
- WO-A-01/89485
- WO-A-90/01933
- WO-A-96/39146
- WO-A-99/15172
- DE-A- 4 424 369
- US-A- 4 990 517
- US-A- 5 223 493
- US-A- 5 607 942
- KRASEMANN, C. (1) ET AL: "Efficacy of moxifloxacin against Staphylococcus aureus in respiratory tract and skin and skin structure infections." JOURNAL OF ANTIMICROBIAL CHEMOTHERAPY, (JULY, 1999) VOL. 44, NO. SUPPL. A, PP. 150. MEETING INFO.: 21ST INTERNATIONAL CONGRESS OF CHEMOTHERAPY BIRMINGHAM, ENGLAND, UK JULY 4-7, 1999, XP000892776
- ELIES W.: "[Novel fluoroquinolones in the treatment of ENT infections]. NEUERE FLUORCHINOLONE BEI DER THERAPIE VON HNO-INFEKTIONEN." CHEMOTHERAPIE JOURNAL, (1998) 7/3 (93-97)., XP000892813

## Description

### Background of the Invention

The present invention is directed to the provision of topical antibiotic pharmaceutical compositions for use in the treatment of ophthalmic, infections commonly associated with *P.aeruginosa.* Such compositions are useful in methods of treating ophthalmic infections by applying those compositions to the affected tissues. The compositions and methods of the invention are based on Moxifloxacin. The compositions of the present invention may also contain one or more anti-inflammatory agents.

The use of quinolone antibiotics to treat infections represents the current state of the art in the field of ophthalmic pharmaceutical compositions and methods of treatment. For example, a topical ophthalmic composition containing the quinolone ciprofloxacin is marketed by Alcon Laboratories, Inc. under the name CILOXAN™ (Ciprofloxacin 0.3%) Ophthalmic Solution. The following quinolones have also been utilized in ophthalmic antibiotic compositions:

| | | |
|---|---|---|
| **Quinolone** | **Product** | **Manufacturer** |
| Ofloxacin | OCUFLOX™ | Allergan |
| Norfloxacin | CHIBROXIN™ | Merck |
| Lomefloxacin | LOMEFLOX™ | Senju |

The foregoing quinolone antibiotic compositions are generally effective in treating ophthalmic infections, and have distinct advantages over prior ophthalmic antibiotic compositions, particularly those having relatively limited spectrums of antimicrobial activity, such as: neomycin, polymyxin B, gentamicin and tobramycin, which are primarily useful against gram negative pathogens; and bacitracin, gramicidin, and erythromycin, which are primarily active against gram positive pathogens. However, despite the general efficacy of the ophthalmic quinolone therapies currently available, there is a need for improved compositions and methods of treatment based on the use of antibiotics that are more effective than existing antibiotics against key ophthalmic pathogens, and less prone to the development of resistance by those pathogens.

WO 9001933 discloses pharmaceutical compositions comprising ciprofloxacin, norfloxacin, ofloxacin, dofloxacin or pefloxacin for the treatment of ophthalmic bacterial infections and inflammation.

Ophthalmic infections are frequently accompanied by inflammation of the infected ophthalmic tissues and perhaps even surrounding tissues. Similarly, ophthalmic surgical procedures that create a risk of microbial infections frequently also cause inflammation of the affected tissues. Thus, there is also a need for ophthalmic pharmaceutical compositions that combine the anti-infective activity of one or more antibiotics with the anti-inflammatory activity of one or more steroid or non-steroid agents in a single composition.

### Summary of the Invention

The invention is based on the use of a potent antibiotic to topically treat ophthalmic infections commonly associated with *P.aeruginosa,* as well as the prophylactic use of this antibiotic following surgery or other trauma to ophthalmic tissues. The compositions of the present invention may also be administered to the affected tissues during ophthalmic surgical procedures to prevent or alleviate post-surgical infection.

The compositions preferably also contain one or more anti-inflammatory agents to treat inflammation associated with infections of ophthalmic tissues. The anti-inflammatory component of the compositions is also useful in treating inflammation associated with physical trauma to ophthalmic tissues, including inflammation resulting from surgical procedures. The compositions of the present invention are therefore particularly useful in treating inflammation associated with trauma to ophthalmic tissues wherein there is either an infection or a risk of an infection resulting from the trauma.

Examples of ophthalmic conditions that may be treated with the compositions of the present invention include conjunctivitis, keratitis, blepharitis, dacyrocystitis, hordeolum and corneal ulcers. The compositions of the invention may also be used prophylactically in connection with various ophthalmic surgical procedures that create a risk of infection.

The compositions of the present invention are specially formulated for topical application to ophthalmic tissues. The compositions are preferably sterile, and have physical properties (e.g., osmolality and pH) that are specially suited for application to ophthalmic tissues, including tissues that have been compromised as the result of preexisting disease, trauma, surgery or other physical conditions.

### Detailed Description of the Invention

The antibiotic used in the compositions and methods of the present invention is Moxifloxacin or a pharmaceutically useful hydrate or salt thereof. Maxifloxacin has the following structure:

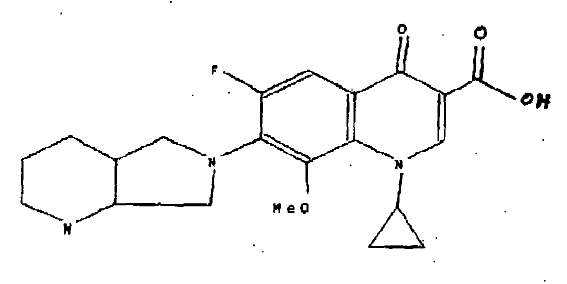

Further details regarding the structure, preparation, and physical properties of Moxifloxacin are provided in United States Patent No. 5,607,942.

The concentrations of Moxiffoxacin or a pharmaceutical useful hydrate or salt thereof in the compositions of the present invention will vary dependent on the intended use of the compositions (e.g., treatment of existing infections or prevention of post-surgical infections), and the relative antimicrobial activity of the specific antibiotic selected. The antimicrobial activity of antibiotics is generally expressed as the minimum concentration required to inhibit the growth of a specified pathogen. This concentration is also referred to as the "minimum inhibitory concentration" or "MIC". The term "MIC90" refers to the minimum concentration of antibiotic required to inhibit the growth of ninety percent (90%) of the strains of a species. The concentration of an antibiotic required to totally kill a specified bacteria is referred to as the "minimum bactericidal concentration" or "MBC". The minimum inhibitory concentration of Moxifloxacin for several bacteria commonly associated with ophthalmic infections are provided in the following table:

| Microorganism | MIC₉₀ |
|---|---|
| S. aureus/methicillin sensitive | 0.13 |
| S. aureus/methicillin resistant | 4.0 |
| S. aureus/quinolone resistant | 4.0 |
| S. epidermidis/methicillin sensitive | 0.25 |
| S. epidermidis/methicillin resistant | 4.0 |
| S. pneumoniae/penicillin sensitive | 0.25 |
| S. pneumoniae/penicillin resistant | 0.25 |
| P. aeruginosa | 8.0 |
| H. influenzae/β-lactamase positive | 0.06 |
| H influenzae/βlactamase negative | 0.06 |

All of the foregoing concentrations are expressed as micrograms per milliliter ("mcg/ml").

The appropriate antibiotic concentration for ophthalmic compositions will generally be an amount of the antibiotic sufficient to provide a concentration in the aqueous humor and lacrimal fluid of the eye equal to or greater than the MIC90 level for the antibiotic relative to gram-negative and gram-positive organisms commonly associated with ophthalmic infections.

Such amounts are referred to herein as "an antimicrobial effective amount". The compositions of the present invention will typically contain Moxifloxacin or a pharmaceutically useful hydrate or salt thereof in a concentration of from about 0.1 to about 1.0 percent by weight ("wt.%") of the compositions.

The compositions of the present invention may also contain one or more anti-inflammatory agents. The anti-inflammatory agents utilized in the present invention are broadly classified as steroidal or non-steroidal. The preferred steroidal anti-inflammatory agents are glucocorticoids.

The preferred glucocorticoids for ophthalmic use include dexamethasone, loteprednol, rimexolone, prednisolone, fluorometholone, hydrocortisone.

The dexamethasone derivatives described in U.S. Patent No. 5,223,493 (Boltralik) are also preferred steroidal anti-inflammatory agents, particularly with respect to compositions for treating ophthalmic inflammation. The following compounds are especially preferred: 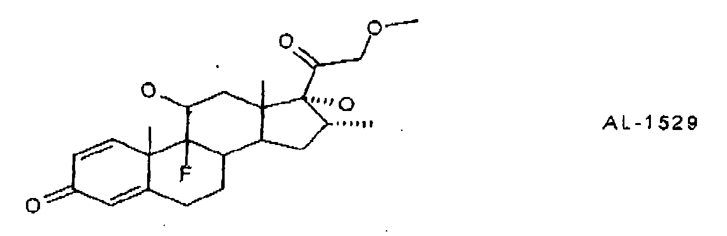 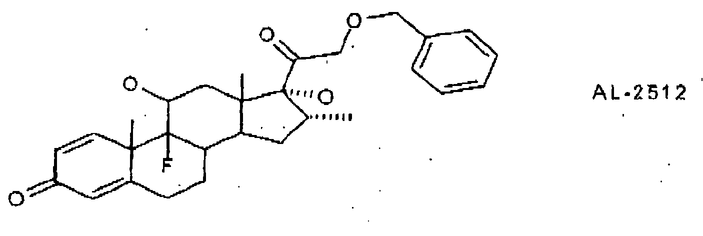 These compounds are referred to herein as "21-ether derivatives of dexamethasone". The 21-benzyl ether derivative (i.e., compound AL-2512) is particularly preferred.

The preferred non-steroidal anti-inflammatory agents are: prostaglandin H synthetase inhibitors (Cox I or Cox II), also referred to as cyclooxygenase type I and type II inhibitors, such as diclofenac, flurbiprofen, ketorolac, suprofen, nepafenac, amfenac, indomethacin, naproxen, ibuprofen, bromfenac, ketoprofen, meclofenamate, piroxicam, sulindac, mefanamic acid, diflusinal, oxaprozin, tolmetin, fenoprofen, benoxaprofen, nabumetome, etodolac, phenylbutazone, aspirin, oxyphenbutazone, NCX-4016, HCT-1026, NCX-284, NCX-456, tenoxicam and carprofen; cyclooxygenase type II selective inhibitors, such as NS-398, vioxx, celecoxib, P54, etodolac, L-804600 and S-33516; PAF antagonists, such as SR-27417, A-137491, ABT-299, apafant, bepafant, minopafant, E-6123, BN-50727, nupafant and modipafant; PDE IV inhibitors, such as ariflo, torbafylline, rolipram, filaminast, piclamilast, cipamfylline, CG-1088, V-I1294A, CT-2820, PD-168787, CP-293121, DWP-205297, CP-220629, SH-636, BAY-19-8004, and roflumilast; inhibitors of cytokine production, such as inhibitors of the NFkB transcription factor; or other anti-inflammatory agents known to those skilled in the art.

The concentrations of the anti-inflammatory agents contained in the compositions of the present invention will vary based on the agent or agents selected and the type of inflammation being treated. The concentrations will be sufficient to reduce inflammation in the targeted ophthalmic tissues following topical application of the compositions to those tissues. Such an amount is referred to herein as "an anti-inflammatory effective amount". The compositions of the present invention will typically contain one or more anti-inflammatory agents in an amount of from about 0.01 to about 1.0 wt.%.

The compositions are typically administered to the affected ophthalmic tissues by topically applying one to four drops of a sterile solution or suspension, or a comparable amount of an ointment, gel or other solid or semisolid composition, one to four times per day. However, the compositions may also be formulated as irrigating solutions that are applied to the affected ophthalmic tissues during surgical procedures.

The ophthalmic compositions of the present invention will contain Moxifloxacin or a pharmaceutically useful hydrate or salt thereof and preferably one or more anti-inflammatory agents, in pharmaceutically acceptable vehicles. The compositions will typically have a pH in the range of 4.5 to 8.0. The ophthalmic compositions must also be formulated to have osmotic values that are compatible with the aqueous humor of the eye and ophthalmic tissues. Such osmotic values will generally be in the range of from about 200 to about 400 milliosmoles per kilogram of water ("mOsm/kg"), but will preferably be about 300 mOsm/kg.

Ophthalmic pharmaceutical products are typically packaged in multidose form. Preservatives are thus required to prevent microbial contamination during use. Suitable preservatives include: polyquaternium-1, benzalkonium chloride, thimerosal, chlorobutanol, methyl paraben, propyl paraben, phenylethyl alcohol, edetate disodium, sorbic acid, or other agents known to those skilled in the art. The use of polyquaternium-1 as the antimicrobial preservative is preferred. Typically such preservatives are employed at a level of from 0.001% to 1.0% by weight.

The solubility of the components of the present compositions may be enhanced by a surfactant or other appropriate co-solvent in the composition. Such co-solvents include polysorbate 20, 60, and 80, polyoxyethylene/polyoxypropylene surfactants (e.g., Pluronic F-68, F-84 and P-103), cyclodextrin, or other agents known to those skilled in the art. Typically such co-solvents are employed at a level of from 0.01% to 2% by weight.

The use of viscosity enhancing agents to provide the compositions of the invention with viscosities greater than the viscosity of simple aqueous solutions may be desirable to increase ocular absorption of the active compounds by the target tissues or increase the retention time in the eye. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents know to those skilled in the art. Such agents are typically employed at a level of from 0.01% to 2% by weight.

The following examples are provided to further illustrate the ophthalmic compositions of the present invention.

### Example 1

| **Ophthalmic** | **Solution** |
|---|---|
| **Ingredient** | **Amount (wt. %)** |
| Moxifloxacin | 0.35 |
| Sodium Acetate | 0.03 |
| Acetic Acid | 0.04 |
| Mannitol | 4.60 |
| EDTA | 0.05 |
| Benzalkonium Chloride | 0.006 |
| Water | q.s. 100 |

### Example 2

| **Ophthalmic** | **Suspension** |
|---|---|
| **Ingredient** | **Amount (wt. %)** |
| Moxifloxacin | 0.3 |
| Dexarnethasone, Micronized USP | 0.10 |
| Benzalkonium Chloride | 0.01 |
| Edetate Disodium, USP | 0.01 |
| Sodium Chloride, USP | 0.3 |
| Sodium Sulfate, USP | 1.2 |
| Tyloxapol, USP | 0.05 |
| Hydroxyethylcellulose | 0.25 |
| Sulfuric Acid and/or | |
| Sodium Hydroxide, NF | q.s. for pH adjustment to 5.5 |
| Purified Water, USP | q.s. to 100 |

### Example 3

| **Ophthalmic Ointment** | |
|---|---|
| **Ingredient** | **Amount (wt.%)** |
| Moxifloxacin | 0.35 |
| Mineral Oil, USP | 2.0 |
| White petrolatium, USP | q.s 100 |

### Example 4

| **Ophthalmic Ointment** | |
|---|---|
| **Ingredient** | **Amount (wt.%)** |
| Moxifloxacin | 0.3 |
| Fluorometholone Azetate, USP | 0.1 |
| Chlorobutanol, Anhydrous, NF | 0.5 |
| Mineral Oil, USP | 5 |
| White Petrotatum, USP | q.s. 100 |

The invention has been described herein by reference to certain preferred embodiments. However, as obvious variations thereon will become apparent to those skilled in the art, the invention is not to be considered as limited thereto.

## Claims

1. Moxifloxacin or a pharmaceutically useful hydrate or salt thereof for use in topical treating or preventing ophthalmic infections commonly associated with P. aeruginosa.

2. Topical ophthalmic composition comprising an antimicrobial effective amount of Moxifloxacin or a pharmaceutically useful hydrate or salt thereof and a pharmaceutically acceptable vehicle therefore for use in treating or preventing ophthalmic infections commonly associated with P. aeruginosa.

3. Composition according to claim 2 comprising Moxifloxacin or a pharmaceutically useful hydrate or salt thereof in a concentration of 0.1 to 1.0 weight-%.

4. Composition according to any one of claims 2 or 3, wherein the treatment or prevention of ophthalmic infections commonly associated with P. aeruginosa comprises topically applying a therapeutically effective amount of the composition to the affected ophthalmic tissue.

5. Composition according to any one of claims 2 to 4, wherein the composition further comprises an anti-inflammatory effective amount of a steroidal or non-steroidal anti-inflammatory agent.

6. Composition according to claim 5, wherein the anti-inflammatory agent comprises a glucocorticoid.

7. Composition according to claim 6, wherein the glucocorticoid is selected from the group consisting of dexamethasone, rimexolone, prednisolone, fluorometholone, hydrocortisone, mometasone, fluticasone, beclomethasone, flunisolide, triamcinolone and budesonide.

8. Composition according to claim 5, wherein the anti-inflammatory agent comprises a non-steroidal agent selected from the group consisting of prostaglandin H synthetase inhibitors, PAF antagonists, and PDE IV inhibitors.

9. Composition according to claim 5, wherein the anti-inflammatory agent comprises dexamethasone.

## Patentansprüche

1. Moxifloxacin oder ein pharmazeutisch verwendbares Hydrat oder Salz davon zur Verwendung bei der topischen Behandlung oder Vorbeugung ophthalmischer Infektionen, die üblicherweise mit P. aeruginosa in Verbindung stehen.

2. Topische ophthalmische Zusammensetzung, umfassend eine antimikrobiell wirksame Menge Moxifloxacin oder eines pharmazeutisch verwendbaren Hydrats oder Salzes davon und ein pharmazeutisch akzeptables Vehikel dafür zur Verwendung bei der Behandlung oder Vorbeugung ophthalmischer Infektionen, die üblicherweise mit P. aeruginosa in Verbindung stehen.

3. Zusammensetzung nach Anspruch 2, umfassend Moxifloxacin oder ein pharmazeutisch verwendbares Hydrat oder Salz davon in einer Konzentration von 0,1 bis 1,0 Gew.-%.

4. Zusammensetzung nach den Ansprüchen 2 oder 3, wobei die Behandlung oder Vorbeugung ophthalmischer Infektionen, die üblicherweise mit P. aeruginosa in Verbindung stehen, das topische Auftragen einer therapeutisch wirksamen Menge der Zusammensetzung auf das betroffene ophthalmische Gewebe umfasst.

5. Zusammensetzung nach einem der Ansprüche 2 bis 4, wobei die Zusammensetzung ferner eine entzündungshemmende wirksame Menge eines steroidalen oder nicht steroidalen Entzündungshemmers umfasst.

6. Zusammensetzung nach Anspruch 5, wobei der Entzündungshemmer ein Glucocorticoid umfasst.

7. Zusammensetzung nach Anspruch 6, wobei das Glucocorticoid aus der Gruppe ausgewählt ist, bestehend aus Dexamethason, Rimexolon, Prednisolon, Fluorometholon, Hydrocortison, Mometason, Fluticason, Beclomethason, Flunisolid, Triamcinolon und Budesonid.

8. Zusammensetzung nach Anspruch 5, wobei der Entzündungshemmer ein nicht steroidales Mittel, ausgewählt aus der Gruppe, bestehend aus Prostaglandin-H-Synthetaseinhibitoren, PAF-Antagonisten und PDE-IV-Inhibitoren, umfasst.

9. Zusammensetzung nach Anspruch 5, wobei der Entzündungshemmer Dexamethason umfasst.

## Revendications

1. Moxifloxacine ou hydrate ou sel de celle-ci à usage pharmaceutique à utiliser dans le traitement local ou la prévention d'infections ophtalmiques habituellement associées au P. aeruginosa.

2. Composition ophtalmique locale comprenant une quantité d'action antimicrobienne de moxifloxacine ou d'un hydrate ou sel de celle-ci à usage pharmaceutique et d'un excipient acceptable pharmaceutiquement à utiliser dans le traitement ou la prévention d'infections ophtalmiques habituellement associées au P. aeruginosa.

3. Composition selon la revendication 2 comprenant de la moxifloxacine ou un hydrate ou sel de celle-ci à usage pharmaceutique dans une concentration de 0,1 à 1,0 % en poids.

4. Composition selon l'une des revendications 2 ou 3 dans laquelle le traitement ou la prévention des infections ophtalmiques habituellement associées au P. aeruginosa comprend l'application locale d'une quantité efficace thérapeutiquement de la composition sur le tissu ophtalmique affecté.

5. Composition selon l'une des revendications 2 à 4 dans laquelle la composition comprend par ailleurs une quantité à action anti-inflammatoire d'un agent anti-inflammatoire stéroïdien ou non-stéroïdien.

6. Composition selon la revendication 5, dans laquelle l'agent anti-inflammatoire comprend un glucocorticoïde.

7. Composition selon la revendication 6, dans laquelle le glucocorticoïde est choisi dans le groupe composé de la dexaméthasone, de la riméxolone, de la prednisolone, de la fluorométholone, de l'hydrocortisone, de la mométasone, de la fluticasone, de la béclométhasone, du flunisolide, de la triamcinolone et du budésonide.

8. Composition selon la revendication 5, dans laquelle l'agent anti-inflammatoire comprend un agent non-stéroïdien choisi dans le groupe composé des inhibiteurs de la prostaglandine H synthétase, les antagonistes de PAF et les inhibiteurs de pDE IV.

9. Composition selon la revendication 5 dans laquelle l'agent anti-inflammatoire comprend la dexaméthasone.
